(19) Europäisches Patentamt
European Patent Office
Office européen des brevets

(11) Publication number: **0 106 335 B1**

# (12) EUROPEAN PATENT SPECIFICATION

(45) Date of publication of patent specification: **23.10.91**

(51) Int. Cl.[5]: **A61K 45/06**, A61K 31/55, //(A61K31/55,31:135), (A61K31/55,31:47),(A61K31/55, 31:445),(A61K31/55,31:40)

(21) Application number: **83110263.7**

(22) Date of filing: **14.10.83**

## (54) Pharmaceutical composition and a method for enhancing the absorption of a pharmaceutically active compound.

(30) Priority: **15.10.82 JP 181940/82**

(43) Date of publication of application: **25.04.84 Bulletin 84/17**

(45) Publication of the grant of the patent: **23.10.91 Bulletin 91/43**

(84) Designated Contracting States: **BE CH DE FR GB IT LI NL SE**

(56) References cited:

**CHEMICAL ABSTRACTS, vol. 96, no. 25, 21st June 1982, page 52, no. 210659e, Columbus, Ohio, US; P. THIEVANT et al.: "Effects of diltiazem on renovascular-hypertensive and on normotensive rats", & GEN. PHARMACOL. 1982, 13(2), 165-8**

(73) Proprietor: **Tanabe Seiyaku Co., Ltd.**
**No. 21 Dosho-machi 3-chome Higashi-ku**
**Osaka-shi Osaka-fu(JP)**

(72) Inventor: **Kohno, Keiichi**
**No. 6-3, Shinsenri Kita-machi 1-chome**
**Toyonaka-shi Osaka-fu(JP)**
Inventor: **Etoh, Akira**
**No. 13-13, Mefuhigashino-cho**
**Takarazuka-shi Hyogo-ken(JP)**
Inventor: **Murata, Kazuo**
**No. 8-4, Segawa 1-chome**
**Minoh-shi Osaka-fu(JP)**
Inventor: **Yamakita, Hirokazu**
**No. 24-6, Shinmori 4-chome Asahi-ku**
**Osaka-shi Osaka-fu(JP)**

(74) Representative: **Hansen, Bernd, Dr.rer.nat. et al**
**Hoffmann, Eitle & Partner Patentanwälte Arabellastrasse 4 Postfach 81 04 20**
**W-8000 München 81(DE)**

EP 0 106 335 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

CHEMICAL ABSTRACTS, vol. 77, no. 1, 3rd July 1972, page 493, no. 5554h, Columbus, Ohio, US; & JP - A - 72 08 544 (TANABE SEYAPUR CO. LTD.) 11-03-1972

EXPERIENTIA, vol. 38, no. 12, December 1982, pages 1468-1469, Basel, CH; M. LIEVRE et al.: "Interaction of diltiazem with propranolol on atrioventricular conduction and refractoriness in the dog"

CHEMICAL ABSTRACTS, vol. 99, no.7, 15th August 1983, page 14, no. 47525j, Columbus, Ohio, US; A. ETOH et al.: "Studies on the drug interaction of diltiazem. III. Mechanism of the absorption enhancing effect of diltiazem on the co-administrated propranolol", & YAKUGAKU ZASSHI 1983, 103(5), 573-80

CHEMICAL ABSTRACTS, vol. 99, no.7, 15th August 1983, page 14, no. 47526k, Columbus, Ohio, US; A. ETOH et al.: "Studies on the drug interaction of diltiazem. IV. Relationship between first pass metabolism of various drugs and the absorption enhancing effect of diltiazem", & YAKUGAKU ZASSHI 1983, 103(5), 581-8

"Goodman and Gilman's-The Pharmacological basis of Therapeutics".6th Ed,(1980)p.1702, 1714, 1721, 1727.

Kornhauser et al. Pharmacol Ther 1978, 23, p.165-174

## Description

This invention relates to a novel pharmaceutical composition and to a method enhancing the absorption of a pharmaceutically active compound. More particularly, it relates to a pharmaceutical composition which comprises a pharmaceutically active compound having an absolute bioavailability of not more than 20 % and diltiazem or a pharmaceutically acceptable acid addition salt thereof.

It is known that diltiazem (chemical name: d-3-acetoxy-cis-2,3-dihydro-5-[2-(dimethylamino)ethyl]-2-(p-methoxyphenyl)-1,5-benzothiazepin-4(5H)-one) or a pharmaceutically acceptable acid addition salt thereof is useful as a calcium-antagonistic coronary vasodilator, but the effect of this compound on the absorption of a co-administered drug has not been known.

Pharmaceutically active compounds (or drugs) may be administered by various routes including oral, rectal, sublingual (buccal), intravenous, intramuscular, subcutaneous and topical routes. Among these routes, the oral route is generally most convenient. However, oral administration of a drug to patients sometimes gives rise to a great deal of inter-individual difference in the blood concentration of the drug because of variation in absorbability of the drug between each patient, while blood concentration of the drug may also be affected by a number of other factors such as, for example, solubility thereof in gastro-intestinal fluids, stability against chemical or enzymatic decomposition, permeability through gastro-intestinal mucosa or metabolic decomposition in the liver. Moreover, bioavailability of a drug is an important factor to elicit the pharmacological effect thereof in the living body. However, when a drug which shows poor bioavailability is administered orally, it is generally difficult to achieve an adequate blood concentration of the drug. As a matter of course, the blood concentration of such drug may be increased to some extent by increasing the dose thereof, but in this case the drug nay cause undesirable side effects.

As a result of various investigations, we have now found that diltiazem or a pharmaceutically acceptable acid addition salt thereof is quite useful to improve or enhance the bioavailability of a pharmaceutically active compound having poor oral bioavailability. Namely, when a pharmaceutically active compound (or drug) has poor bioavailability (i.e., an absolute bioavailability of not more than 20 %), the absorption of said pharmaceutically active compound can be remarkably enhanced by administering it enterally together with diltiazem or a pharmaceutically acceptable acid addition salt thereof.

An object of the present invention is to provide a pharmaceutical composition suitable for use by enteral administration (e.g., oral or rectal administration) and also suitable for enhancing absorption of a pharmaceutically active compound having an absolute bioavailability of not more than 20 %. Another object of the invention is to provide a pharmaceutical composition comprising a pharmaceutical active compound having an absolute bioavailability of not more than 20 % and diltiazem or a pharmaceutically acceptable acid addition salt thereof. A further object of the invention is to provide a method for enhancing absorption of a pharmaceutically active compound by administering it enterally together with diltiazem or a pharmaceutically acceptable acid addition salt thereof. These and other objects of the present invention will be apparent to persons skilled in the art from the following description.

In this specification and claims, the terms "absolute bioavailability" is defined as a value which is calculated by the following formula:

$$\frac{\text{AUC p.o.}}{\text{AUC i.v.}} \times \frac{\text{Dose i.v.}}{\text{Dose p.o.}} \times 100$$

wherein "AUC p.o." and "AUC i.v." stand for the areas under the blood concentration-time curves which are estimated by oral and intravenous administration of a drug. The smaller the above-mentioned value, the less the amount of a drug which reaches the systemic circulation. Therefore, the term "absolute bioavailability" always shows the degree of completeness of absorption of a pharmaceutically active compound in systemic circulation.

The absorption-enhancing effect of diltiazem is observed in any pharmaceutically active compound having an absolute bioavailability of not more than 20 %. Thus, a wide variety of pharmaceutically active compounds having such absolute bioavailabilitycan be used for the purpose of the present invention irrespectively of the pharmacological or therapeutic effects thereof, insofar as they are not contraindicated with diltiazem.

Examples of such pharmaceutically active compounds include:

(i) adrenergic β-blocking agents such as alprenolol [chemical name: 1-(2-allylphenoxy)-3-(isopropylamino)-2-propanol];

(ii) catecholamines acting on the sympathetic nervous system such as dopamine, N-L-alanyldopamine

[chemical name: N-(L-alanyl)-3,4-dihydroxyphenethylamine], N-L-isoleucyldopamine [chemical name: N-(L-isoleucyl)-3,4-dihydroxyphenethylamine], N-(N-acetylalanyl)-3,4-diethoxycarboxyphenethylamine, N-(N-acetylmethionyl)-3,4-diethoxycarboxyphenethylamine or isoproterenol;

(iii) benzodiazepine derivatives acting on central nervous system such as flurazepam [chemical name: 7-chloro-1-[2-dimethylamino)ethyl]-5-(o-fluorophenyl)-1,3-dihydro-2H-1,4-benzodiazepin-2-one];

(iv) vasodilators such as amyl nitrite;

(v) cardiotonics or antidiabetic agents such as benzylalcohol derivatives of the formula:

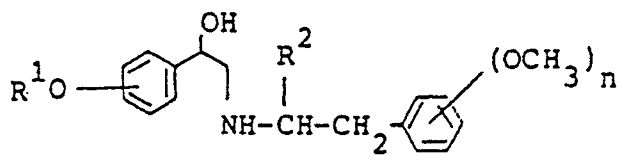

wherein $R^1$ is hydrogen or alkyl of one to 4 carbon atoms, $R^2$ is hydrogen or alkyl of one to 4 carbon atoms and n is an integer of one to 3;

(vi) bronchodilators such as tetrahydroisoquinoline derivatives of the formula:

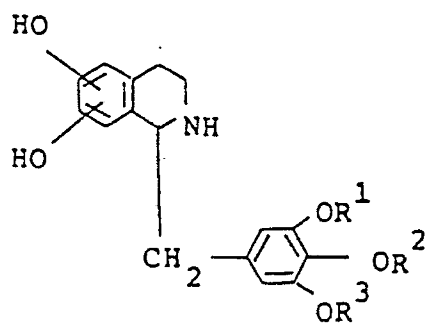

wherein each of $R^1$, $R^2$ and $R^3$ is hydrogen or alkyl of one to 4 carbon atoms;

(vii) hemostatics such as carbazochrome sulfonic acid [chemical name: 1-methyl-5-semicarbazono-6-oxo-2,3,5,6-tetrahydroindole-2-sulfonic acid] or adrenochrome monoaminoguanizine;

(viii) antispasmodics such as timepidium halide [chemical name: 1,1-dimethyl-5-methoxy-3-(dithien-2-ylmethylene)-piperidinium halide]; and

(ix) antitussives such as tipepidine [chemical name: 3-(di-2-thienylmethylene)-1-methylpiperidine].

The above-mentioned drugs may be used either in free form or in the form of pharmaceutically acceptable salts thereof. Depending on the physico-chemical characteristics of the drug, said pharmaceutically acceptable salt may further be either inorganic acid addition salts such as hydrochloride, hydrobromide or sulfate; organic acid addition salts such as citrate, acetate, oxalate, hibenzate (i.e., 2-(4-hydroxybenzoyl)benzoate) or methanesulfonate; alkali metal salts such as sodium salt or potassium salt; alkaline earth metal salts such as calcium salt or magnesium salt and amine salts.

Among the above-mentioned drugs, especially suitable for use in the present invention are alprenolol, dopamine, N-(N-acetylalanyl)-3,4-diethoxycarboxyphenethylamine, N-(N-acetylmethionyl)-3,4-diethoxycarboxyphenethylamine, α-(3,4-dimethoxyphenethylaminomethyl)-4-hydroxybenzylalcohol, α-(3,4-dimethoxyphenethylaminomethyl)-3-hydroxybenzylalcohol, α-[(α-methyl-3,4,5-trimethoxyphenethylamino)methyl]-2-hydroxybenzylalcohol, trimetoquinol [chemical name: 1-(3,4,5-trimethoxybenzyl-6,7-dihydroxy-1,2,3,4-tetrahydroisoquinoline], 1-(3,4,5-trimethoxybenzyl)-5,7-dihydroxy-1,2,3,4-tetrahydroisoquinoline, 1-methyl-5-semicarbazono-6-oxo-2,3,5,6-tetrahydroindole-2-sulfonic acid, timepidium halide, tipepidine and pharmaceutically acceptable salts thereof.

On the other hand, diltiazem to be used together with the pharmaceutically active compound may be used in the form of either free base or a pharmaceutically acceptable acid addition salt thereof. Examples of the pharmaceutically acceptable acid addition salt of diltiazem include inorganic acid addition salts such as hydrochloride, hydrobromide, sulfate, nitrate or perchlorate; and organic acid addition salts such as acetate, oxalate, malonate, tartrate, citrate, lactate or aspartate.

The dose of the pharmaceutically active compound to be used together with diltiazem or an acid

addition salt thereof is not critical, but is preferably used within its usual dosage range. For example, alprenolol or its salts at a dose of 50 to 200 mg/day/body; dopamine or its salts at a dose of 200 to 1,000 mg/day/body; N-(N-acetylalanyl)-3,4-diethoxycarboxyphenethylamine at a dose of 500 to 3,000 mg/day/body; N-(N-acetylmethionyl)-3,4-diethoxycarboxyphenethylamine at a dose of 500 to 3,000 mg/day/body; α-(3,4-dimethoxyphenethylaminomethyl)-4-hydroxybenzylalcohol or its salts at a dose of 5 to 50 mg/day/body; α-(3,4-dimethoxyphenethylaminomethyl)-3-hydroxybenzylalcohol or its salts at a dose of 4 to 60 mg/day/body; α-[(α-methyl-3,4,5-trimethoxyphenethylamino)methyl]-2-hydroxybenzylalcohol or its salts at a dose of 5 to 60 mg/day/body; trimethoquinol or its salts at a dose of 4 to 12 mg/day/body; 1-(3,4,5-trimethoxybenzyl)-5,7-dihydroxy-1,2,3,4-tetrahydroisoquinoline or its salts at a dose of 2 to 12 mg/day/body; carbazochrome sulfonic acid or its salts at a dose of 30 to 90 mg/day/body; timepidium halide at a dose of 50 to 100 mg/day/body; and tipepidine or its salts at a dose of 60 to 150 mg/day/body, respectively.

On the other hand, diltiazem or a pharmaceutically acceptable acid addition salt thereof to be used for enhancing the absorption of the pharmaceutically active compound is preferably used at a dose of 5 to 500 mg/day/body, especially 20 to 360 mg/day/body.

The pharmaceutically active compound having an absolute oral bioavailability of not more than 20 % and diltiazem or a pharmaceutically acceptable acid addition salt thereof should preferably be used within the weight ratio of from 1 : 200 to 100 : 1, especially from 1 : 100 to 50 : 1, more especially from 2 : 100 to 20 : 1. For example, the preferred weight ratio of said pharmaceutically active compound to diltiazem or a salt thereof can be shown illustratively as follows: from 0.1 : 1 to 3.0 : 1, especially from 0.2 : 1 to 2 : 1 for alprenolol or its salts; from 2 : 1 to 40 : 1, especially from 2 : 1 to 10 : 1 for dopamine or its salts; from 1 : 1 to 100 : 1, especially from 1 : 1 to 25 : 1 for N-(N-acetylalany)-3,4-diethoxycarboxyphenethylamine; from 1 : 1 to 100 : 1, especially from 1 : 1 to 25 : 1 for N-(N-acetylmethionyl)-3,4-diethoxycarboxyphenethylamine; from 0.1 : 1 to 1 : 1, especially from 0.14 : 1 to 0.5 : 1 for α-(3,4-dimethoxyphenethylaminomethyl)-4-hydroxybenzylalcohol or its salts; from 0.12 : 1 to 0.8 : 1, especially from 0.17 : 1 to 0.5 : 1 for α-(3,4-dimethoxyphenethylaminomethyl)-3-hydroxybenzylalcohol or its salts; from 0.12 : 1 to 1 : 1, especially from 0.17 : 1 to 0.5 : 1 for α-[(α-methyl-3,4,5-trimethoxyphenethylamino)methyl]-2-hydroxybenzylalcohol or its salts; from 0.02 : 1 to 0.8 : 1, especially from 0.03 : 1 to 0.2 : 1 for trimethoquinol or its salts; from 0.024 : 1 to 0.4 : 1, especially from 0.03 : 1 to 0.1 : 1 for 1-(3,4,5-trimethoxybenzyl)-5,7-dihydroxy-1,2,3,4-tetrahydroisoquinoline or its salts; from 0.2 : 1 to 6 : 1, especially from 0.25 : 1 to 1.5 : 1 for carbazochrome alkali metal sulfonate; from 0.18 : 1 to 18 : 1, especially from 0.25 : 1 to 4.5 : 1 for timepidium halide; and from 0.3 : 1 to 14 : 1, especially from 0.4 : 1 to 4 : 1 for tipepidine or its salts.

The pharmaceutically active compound having an absolute oral bioavailability of not more than 20 % and diltiazem or a pharmaceutically acceptable acid addition salt thereof may be administered to a warm-blooded animal including man through conventional enteral route, for example, by oral route or rectal route. Further, these components may be administered in the form of a conventional preparation, for example, in a solid dosage form such as tablets, pills, powders, granules or suppositories; or in a liquid dosage form such as solutions, syrups, emulsions, elixirs, suspensions or lemonades. In formulating these preparations, there may be used a pharmaceutically acceptable carrier or diluent such as binders (e.g., syrup, arabic gum, gelatin, sorbit, tragacanth, polyvinylpyrrolidone), diluents (lactose, sucrose, corn starch, calcium phosphate, sorbit), lubricants (e.g., magnesium stearate, talc, polyethylene glycol, silica), disintegrators (e.g., corn starch), wetting agents (e.g., sodium lauryl sulfate) and suppository bases (e.g., cacao butter, laurin butter, polyethylene glycol (e.g., macrogol), glycerinated gelatin, triglyceride of saturated fatty acids ($C_{12}$ - $C_{18}$) (e.g., Witepsol®)). Flavoring agents, sweetening agents or coloring agents may also be added thereto.

The pharmaceutical composition of the present invention may be in the form of a single preparation containing both the pharmaceutically active compound and diltiazem or its salt. Alternatively, each components of the invention, i.e., the pharmaceutically active compound and diltiazem or its salt, may be formulated into separate preparations to be used simultaneously for patients, e.g., into a unit-dosage-pack containing each components separately.

Diltiazem or a pharmaceutically acceptable acid addition salt thereof can enhance the absorption of a pharmaceutically active compound insofar as said pharmaceutically active compound is the one having an absolute bioavailability of not more than 20 %, and hence, can increase the concentration of said pharmaceutically active compound in blood. Therefore, diltiazem or its salts when administered enterally together with the pharmaceutically active compound can improve the bioavailability of said pharmaceutically active compound and can also reduce doses of said co-administered active compound. The simultaneous administration of the pharmaceutically active compound and diltiazem or its salts is further advantageous to reduce inter-individual variation in the pharmacological effect of the pharmaceutically active compound, whereas oral administration of the pharmaceutically active compound sometimes brings about large variation in the pharmacological effects thereof between each patients due to difference in the bioavailability

thereof. Further, the simultaneous administration of α-(3,4-dimethoxyphenethylaminomethyl)-4-hydroxybenzylalcohol (cardiotonic agent) and diltiazem is advantageous for treatment of patients without undesirable effects on heart rate because a possible increase in heart rate caused by the former compound is compensated by diltiazem.

The present invention is illustrated by the following Experiments and Examples.

Experiment 1

Male beagle dogs (body weight: 12 to 14 kg; one group: 3 to 8 beagle dogs) fasted for 24 hours were used in each groups. A drug having an absolute bioavailability of not more than 20 % and diltiazem hydrochloride were administered orally to the beagle dogs. After one week intervals, the drug only was administered orally to the same beagle dogs. The plasma drug concentration was examined at intervals by TCL densito metry if the drug was timepidium bromide; or by high-peformance liquid chromatography if the drug was tipepidine hibenzate or carbazochrome sodium sulfate. Based on the results obtained above, the absorption enhancing effect of diltiazem hydrochloride was calculated by the following formula;

$$\text{Absorption enhacing effect} = \frac{\text{Area under the plasma drug concentration-time curve estimated by co-administration of the drug and diltiazem hydrochloride}}{\text{Area under the plasma drug concentration-time curve estimated by administration of the drug only}}$$

The results are shown in the following Table 2.

Table 2

| Drug and its dose | Dose of diltiazem hydrochloride | Absorption enhancing effect |
|---|---|---|
| Timepidium bromide (300 mg) | 30 mg | 2.3 |
| Tipepidine hibenzate (50 mg) | 60 mg | 2.1 |
| Carbazochrome sodium sulfonate (100 mg) | 60 mg | 1.9 |

Example 1

| (Capsules) | |
|---|---|
| alprenolol hydrochloride | 15.0 g |
| diltiazem hydrochloride | 60.0 g |
| starch | 20.0 g |
| lactose | 194.0 g |
| polyvinylpyrrolidone | 10.0 g |
| magnesium stearate | 0.7 g |
| hydrated silicic acid | 0.3 g |
| | Total 300 g |

A mixture of alprenolol hydrochloride, diltiazem hydrochloride, starch and lactose is added to an ethanol solution of polyvinylpyrrolidone, and the mixture is kneaded, granulated and then dried. Magnesium stearate and hydrated silicic acid are added to the dried granules, and the mixture is filled into capsules in an amount of 300 mg per capsule.

Example 2

| (Tablets) | |
|---|---|
| trimetoquinol hydrochloride | 3.0 g |
| diltiazem hydrochloride | 50.0 g |
| lactose | 115.0 g |
| polyvinylpyrrolidone | 6.0 g |
| carboxymethylcellulose calcium | 24.5 g |
| magnesium stearate | 1.5 g |
| | Total 200 g |

A mixture of trimetoquinol hydrochloride, diltiazem hydrochloride and lactose is added to an ethanol solution of polyvinylpyrrolidone, and the mixtrue is kneaded, granulated and then dried. Magnesium stearate and carboxymethylcellulose calcium are added to the dried granules, and the mixture is compressed into tablets of 8 mm in diameter (average weight per each tablets: 200 mg).

Example 3

| (Granules for making syrups) | |
|---|---|
| tipepidine hibenzate | 2.0 g |
| diltiazem citrate | 2.3 g |
| anhydrous glucose | 70.0 g |
| lactose | 23.0 g |
| hydroxypropylcellulose | 1.0 g |
| monosodium fumarate | 1.0 g |
| hydrated silicic acid | 0.3 g |
| flavors | a sufficient quantity |
| | Total 100 g |

A mixture of tipepidine hibenzate, diltiazem citrate, anhydrous glucose, lactose and monosodium fumarate is added to an ethanol solution of hydroxypropylcellulose, and the mixture is kneaded, granulated and then dried. Hydrated silicic acid and flavors are added to the dried granules. The thus-obtained granules are used to make syrups.

Example 4

| (Suppositories) | |
|---|---|
| N-(N-acetylmethionyl)-3,4-dimethoxycarboxyphenethylamine | 200.0 g |
| diltiazem hydrochloride | 60.0 g |
| triglyceride of fatty acid ($C_{12}$ - $C_{18}$) (i.e., Witepsol W-35) | 1340 g |
| | Total 1600 g |

Witepsol W-35 is melted at 60° C and then cooled to 40 ° C. N-(N-acetylmethionyl)-3,4-diethoxycarboxyphenethylamine and diltiazem hydrochloride are added to the Witepsol W-35, and the mixture is blended homogeneously for about 20 minutes. The resultant mixture is poured into a suppository mold and then allowed to stand at room temperature to give suppositories (average weight of each suppositories: 1.6 g) each containing 200 mg of N-(N-acetylmethionyl)-3,4-diethoxycarboxyphenethylamine and 60 mg of diltiazem hydrochloride.

Example 5

| (Tablets) | |
|---|---|
| ℓ-1-(3,4,5-trimethoxybenzyl)-5,7-dihydroxy-1,2,3,4-tetrahydroisoquinoline hydrochloride | 3.0 g |
| diltiazem hydrochloride | 50.0 g |
| lactose | 115.0 g |
| polyvinylpyrrolidone | 6.0 g |
| carboxymethylcellulose calcium | 24.5 g |
| magnesium stearate | 1.5 g |
| | Total 200 g |

A mixture of ℓ-1-(3,4,5-trimethoxybenzyl)-5,7-dihydroxy-1,2,3,4-tetrahydroisoquinoline hydrochloride, diltiazem hydrochloride and lactose is added to an ethanol solution of polyvinylpyrrolidone, and the mixture is kneaded, granulated and then dried. Magnesium stearate and carboxymethylcellulose calcium are added to the dried granules, and the mixture is compressed into tablets of 8mm in diameter (average per each tablets: 200 mg).

Example 6

| (Capsules) | |
|---|---|
| N-(N-acetylmethionyl)-3,4-diethoxycarboxyphenethylamine | 200.0 g |
| diltiazem hydrochloride | 160.0 g |
| starch | 130.0 g |
| polyvinylpyrrolidone | 15.0 g |
| magnesium stearate | 5.0 g |
| | Total 510 g |

A mixture of N-(N-acetylmethionyl)-3,4-diethoxycarboxyphenethylamine, diltiazem hydrochloride and starch is added to an ethanol solution of polyvinylpyrrolidone, and the mixture is kneaded, granulated and then dried. Magnesium stearate is added to the dried granules, and the mixture is filled into capsules in an amount of 510 mg per capsule.

Example 7

| (Capsules) | |
|---|---|
| N-(N-acetylalanyl)-3,4-diethoxycarboxyphenethylamine | 200.0 g |
| diltiazem hydrochloride | 160.0 g |
| starch | 130.0 g |
| polyvinylpyrrolidone | 15.0 g |
| magnesium stearate | 5.0 g |
| | Total 510 g |

A mixture of N-(N-acetylalanyl)-3,4-diethoxycarboxyphenethylamine, diltiazem hydrochloride and starch is added to an ethanol solution of polyvinylpyrrolidone, and the mixture is kneaded, granulted and then dried. Magnesium stearate is added to the dried granules, and the mixture is filled into capsules in an amount of 510 mg per capsule.

Example 8

| (Capsules) | |
|---|---|
| dopamine hydrochloride | 300.0 g |
| diltiazem hydrochloride | 100.0 g |
| starch | 79.0 g |
| polyvinylpyrrolidone | 16.0 g |
| magnesium stearate | 5.0 g |
| | Total 500 g |

A mixture of dopamine hydrochloride, diltiazem hydrochloride and starch is added to an ethanol solution of polyvinylpyrrolidone, and the mixture is kneaded, granulated and then dried. Magnessium stearate is added to the dried graules, and the mixture is filled into capsules in an amount of 500 mg per capsule.

Example 9

| (Tablets) | |
|---|---|
| α-(3,4-dimethoxyphenethylaminomethyl)-4-hydroxybenzylalcohol | 100.0 g |
| diltiazem hydrochloride | 250.0 g |
| starch | 50.0 g |
| crystalline cellulose | 792.0 g |
| magnesium stearate | 8.0 g |
| | Total 1200 g |

The above-mentioned ingredients are mixed, and the mixture is compressed into tablets of 7 mm in diameter (average weight per each tablets: 120 mg).

Example 10

| (Tablets) | |
|---|---|
| α-(3,4-dimethoxyphenethylaminomethyl)-3-hydroxybenzylalcohol | 100.0 g |
| diltiazem hydrochloride | 250.0 g |
| starch | 50.0 g |
| crystalline cellulose | 792.0 g |
| magnesium stearate | 8.0 g |
| | Total 1200 g |

The above-mentioned ingredients are mixed, and the mixture is compressed into tablets of 7 mm in diameter (aveage weight per each tablets: 120 mg).

Example 11

| (Tablets) | |
|---|---|
| α-[(α-methyl-3,4,5-trimethoxyphenethylamino)methyl]-2-hydroxybenzylalcohol | 100.0 g |
| diltiazem hydrochloride | 250.0 g |
| starch | 50.0 g |
| crystalline cellulose | 792.0 g |
| magnesium stearate | 8.0 g |
| | Total 1200 g |

The above-mentioned ingredients are mixed, and the mixture is compressed into tablets of 7 mm in

diameter (average weight per each tablets: 120 mg).

Example 12

| (Capsules) | |
|---|---|
| carbazochrome sodium sulfonate<br>diltiazem hydrochloride<br>crystalline cellulose<br>magnesium stearate | 30.0 g<br>60.0 g<br>207.0 g<br>3.0 g<br>Total 300 g |

The above-mentioned ingredients are mixed, and the mixture is filled into capsules in an amount of 300 mg per capsule.

**Claims**

1. A pharmaceutical composition which comprises a pharmaceutically active compound having an absolute bioavailability of not more than 20 %, and diltiazem or a pharmaceutically acceptable acid addition salt thereof; the weight ratio of the pharmaceutically active compound to diltiazem or a pharmaceutically acceptable acid addition salt thereof being from 1 : 200 to 100 : 1.

2. A pharmaceutical composition according to claim 1, wherein the pharmaceutically active compound is selected from alprenolol, dopamine, N-L-alanyldopamine, N-L-isoleucyldopamine, N-(N- acetylalanyl)-3, 4-diethoxycarboxyphenethylamine, N-(N-acetylmethionyl)-3, 4-diethoxycarboxyphenethylamine, isoproterenol, flurazepam, amyl nitrite, a benzylalcohol derivative of the formula:

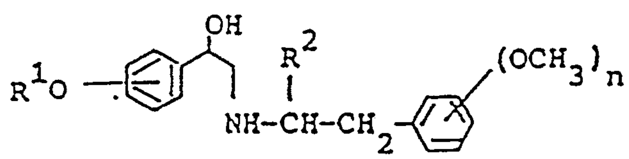

wherein $R^1$ is hydrogen or alkyl of one to 4 carbon atoms, $R^2$ is hydrogen or alkyl of one to 4 carbon atoms and n is an integer of one to 3, a tetrahydroisoquinoline derivative of the formula:

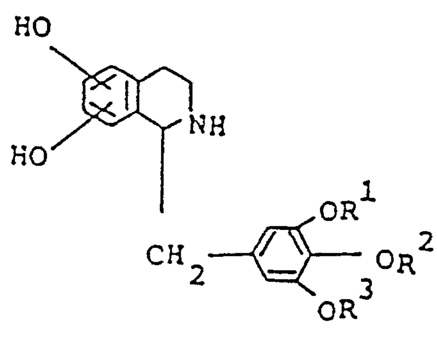

wherein each of $R^1$, $R^2$ and $R^3$ is hydrogen or alkyl of one to 4 carbon atoms, 1-methyl-5-semicarbazono-6-oxo-2,3,5,6-tetrahydroindole-2-sulfonic acid, adrenochrome monoaminoguanizine, timepidium halide, tipepidine and pharmaceutically acceptable salts thereof.

3. A pharmaceutical composition according to claim 1, wherein the pharmaceutically active compound is selected from alprenolol, dopamine, N-(N-acetylalanyl)-3,4-diethoxycarboxyphenethylamine, N-(N-acetylmethionyl)-3,4-diethoxycarboxyphenethylamine, α-(3,4-dimethoxyphenethylaminomethyl)-4-hydroxybenzylalcohol, α-(3,4-dimethoxyphenethylaminomethyl)-3-hydroxybenzylalcohol, α-[(α-methyl-3,4,5-trimethoxyphenethylamino)methyl] -2-hydroxybenzylalcohol, trimetoquinol, 1-(3,4,5-trimethoxybenzyl)-5,7-dihydroxy-1,2,3,4-tetrahydroisoquinoline,1-methyl-5-semicarbazono-6-oxo-2,3,5,6-tetrahydroindole-2-sulfonic acid, timepidium halide, tipepidine and pharmaceutically acceptable salts thereof.

4. A pharmaceutical composition according claim 1, which is in a dosage form suitable for oral administration.

5. A pharmaceutical composition according to claim 1, which is in a dosage form suitable for rectal administration.

## Revendications

1. Composition pharmaceutique qui comprend un composé pharmaceutiquement actif, ayant une biodisponibilité absolue non supérieure à 20 %, et du diltiazem ou un sel d'addition d'acide pharmaceutiquement acceptable de ce composé ; le rapport pondéral entre le composé pharmaceutiquement actif et le diltiazem ou un sel d'addition pharmaceutiquement acceptable de diltiazem, situant entre 2:200 et 100:1.

2. Composition pharmaceutique selon la revendication 1, dans laquelle le composé pharmaceutiquement actif est choisi parmi l'alprénonol, la dopamine, la N-L-alanyldopamine, le N-L-isoleucyldopamine, la N-(N-acétylalanyl)3,4-diéthoxycarboxyphénéthylamine, la N-(N-acétylméthionyl)-3,4-diéthoxycarboxyphénéthylamine, l'isoprotérénol, le flurazépam, le nitrite d'amyle, un dérivé d'alcool benzylique de formule :

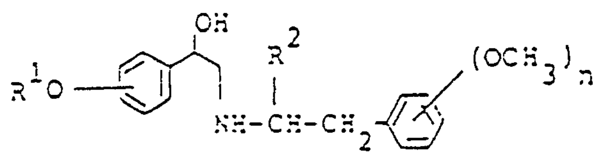

dans laquelle $R^1$ représente un atome d'hydrogène ou un groupe alkyle ayant 1 à 4 atomes de carbone ; $R^2$ représente un atome d'hydrogène ou un groupe alkyle ayant 1 à 4 atomes de carbone ; et n est un nombre entier valant 1 à 3 ; un dérivé de tétraisoquinoléine de formule :

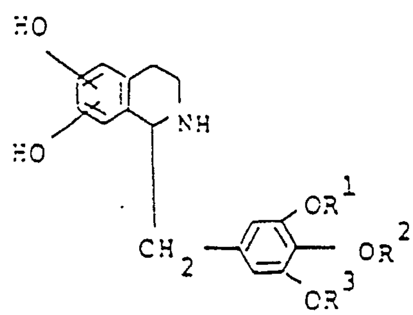

dans laquelle chacun des symboles $R^1$, $R^2$ et $R^3$ représente un atome d'hydrogène ou un groupe alkyle ayant 1 à 4 atomes de carbone, l'acide 1-méthyl-5-semicarbazono-6-oxo-2,3,5,6-tétrahydroindole-2-sulfonique, l'adrénochrome monoaminoquanizine, un halogénure de timépidium, la tipépidine et un de leur sel pharmaceutiquement acceptable.

3. Composition pharmaceutique selon la revendication 1, dans laquelle le composé pharmaceutiquement actif est choisi parmi l'alprénonol, la dopamine, la N-(N-acétylalanyl)-3,4-diéthoxycarboxyphénéthylamine, la N-(N-acétylméthionyl)-3,4-diéthoxycarboxyphénéthylamine, l'alcool α-(3,4-diméthoxyphénéthylaminométhyl)-4-hydroxybenzylique, l'alcool α-(3,4-diméthoxyphénéthylaminoéthyl)-3-hydroxybenzylique, l'alcool α-[(α-méthyl-3,4,5-triméthoxyphénéthylamino)méthyl]-2-hydroxybenzylique, le trimétoquinol (nom chimique : 1-(3,4,5-triméthoxybenzyl-6,7-dihydroxy-1,2,3,4-tétrahydroisoquinoléine], la 1-(3,4,5-triméthoxybenzyl)-5,7-dihydroxy-1,2,3,4-tétrahydroisoquinoléine,

l'acide 1-méthyl-5-semicarbazono-6-oxo-2,3,4,5,6-tétrahydroindole-2-sulfonique, un halogénure de timépidium, la tipépidine et leurs sels pharmaceutiquement acceptables

4. Composition pharmaceutique selon la revendication 1, qui est sous une forme dosée convenant pour une administration par voie orale.

5. Composition pharmaceutique selon la revendication 1, qui est sous une forme dosée convenant pour une administration rectale.

**Patentansprüche**

1. Pharmazeutische Zusammensetzung, umfassend eine pharmazeutisch wirksame Verbindung mit einer absoluten Bioverfügbarkeit von nicht mehr als 20 % und Diltiazem oder ein pharmazeutisch verträgliches Säureadditionssalz hiervon, wobei das Gewichtsverhältnis der pharmazeutisch wirksamen Verbindung zu Diltiazem oder dem pharmazeutisch verträglichen Säureadditionssalz hiervon bei 1:200 bis 100:1 liegt.

2. Pharmazeutische Zusammensetzung nach Anspruch 1, wobei die pharmazeutisch wirksame Verbindung aus Alprenolol, Dopamin, N-L-Alanyldopamin, N-L-Isoleucyldopamin, N-(N-Acetylalanyl)-3,4-diethoxycarboxyphenethylamin, N-(N-Acetylmethionyl)-3,4-diethoxycarboxyphenethylamin, Isoproterenol, Flurazepam, Amylnitrit, einem Benzylalkoholderivat der Formel

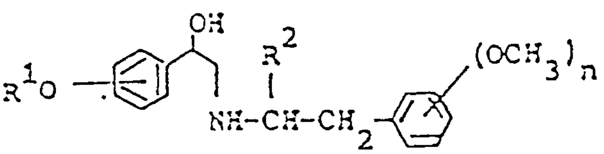

wobei $R^1$ Wasserstoff oder eine Alkylgruppe mit 1 bis 4 Kohlenstoffatomen ist, $R^2$ Wasserstoff oder eine Alkylgruppe mit 1 bis 4 Kohlenstoffatomen ist, und n eine Zahl von 1 bis 3 darstellt, einem Tetrahydroisochinolinderivat der Formel

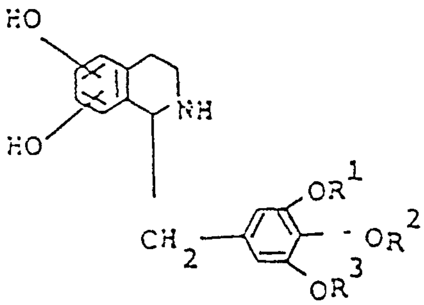

wobei jeweils $R^1$, $R^2$ und $R^3$ Wasserstoff oder eine Alkylgruppe mit 1 bis 4 Kohlenstoffatomen ist, 1-Methyl-5-semicarbazono-6-oxo-2,3,5,6-tetrahydroindol-2-sulfonsäure, Adrenochrommonoaminoguanizin, Timepidiumhalogenid, Tipepidin und pharmazeutisch verträglichen Salzen hiervon ausgewählt ist.

3. Pharmazeutische Zusammensetzung nach Anspruch 1, wobei die pharmazeutisch wirksame Verbindung aus Alprenolol, Dopamin, N-(N-Acetylalanyl)-3,4-diethoxycarboxyphenethylamin, N-(N-Acetylmethionyl)-3,4-diethoxycarboxyphenethylamin, alpha-(3,4-Dimethoxyphenethylaminomethyl)-4-hydroxybenzylalkohol, alpha-(3,4-Dimethoxyphenethylaminomethyl)-3-hydroxybenzylalkohol, alpha-[(alpha-Methyl-3,4,5-trimethoxyphenethylamino)methyl]-2-hydroxybenzylalkohol, Trimetochinol, 1-(3,4,5-Trimethoxybenzyl)-5,7-dihydroxy-1,2,3,4-tetrahydroisochinolin, 1-Methyl-5-semicarbazono-6-oxo-2,3,5,6-tetrahydroindol-2-sulfonsäure, Timepidiumhalogenid, Tipepidin und pharmazeutisch verträglichen Salzen hiervon ausgewählt ist.

**4.** Pharmazeutische Zusammensetzung nach Anspruch 1, die in einer Dosierungsform, die für eine orale Verabreichung geeignet ist, vorliegt.

**5.** Pharmazeutische Zusammensetzung nach Anspruch 1, die in einer Dosierungsform, die für eine rektale Verabreichung geeignet ist, vorliegt.